(19) Europäisches Patentamt
European Patent Office
Office européen des brevets

(11) Numéro de publication: **0 015 798**
**B1**

(12) # FASCICULE DE BREVET EUROPEEN

(45) Date de publication du fascicule du brevet: **24.03.82**

(21) Numéro de dépôt: **80400210.3**

(22) Date de dépôt: **12.02.80**

(51) Int. Cl.³: **C 07 D 513/04,**
A 61 K 31/425,
A 61 K 31/505
//(C07D513/04, 277/00,
235/00), (C07D513/04,
277/00, 239/00)

(54) **Dérivés de thiazole, leur préparation et médicaments les contenant.**

(30) Priorité: **12.02.79 FR 7903431**

(43) Date de publication de la demande:
**17.09.80 Bulletin 80/19**

(45) *Mention de la délivrance du brevet:*
**24.03.82 Bulletin 82/12**

(84) Etats contractants désignés:
**AT BE CH DE FR GB IT LU NL SE**

(56) Documents cités:
FR - A - 2 014 048
FR - A - 2 223 371

(73) Titulaire: **SYNTHELABO**
**58 rue de la Glacière**
**F-75013 Paris (FR)**

(72) Inventeur: **Bigg, Denis**
**5 parc de Diane**
**F-78350 Jouy en Josas (FR)**

(74) Mandataire: **Casanova, André, et al,**
**CABINET ARMENGAUD JEUNE CASANOVA, AKERMAN, LEPEUDRY 23 boulevard de Strasbourg**
**F-75010 Paris (FR)**

**EP 0 015 798 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Dérivés de thiazole, leur preparation et médicaments les contenant

La présente invention concerne des dérivés de thiazole, leurs sels d'addition aux acides pharmaceutiquement acceptables, leur préparation et leur application en thérapeutique.

On connaît le brevet FR—2 014 048 ayant pour objet des dérivés de thiazole qui possèdent principalement des propriétés anorexigènes et subsidiairement des propriétés antidépressives. Ces composés comportent en position 3 du cycle thiazole un radical phényle directement lié au cycle, ainsi qu'un radical $R_1$ = alkyle en position 2. Or, la présente invention propose de nouveaux dérivés du thiazole comportant un groupe phénoxymethyle en position 3 et non substitués en position 2 du thiazole, dérivés qui sont des antidépresseurs.

Les composés de l'invention répondent à la formule (I)

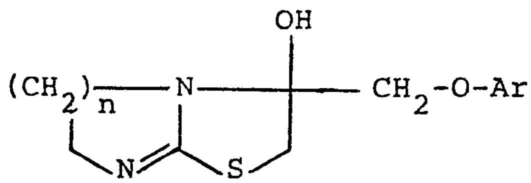

(I)

dans laquelle n est 1 ou 2 et Ar est un radical phényle pouvant porter un ou plusieurs substituants choisis dans le groupe constitué par les atomes d'halogène, $CF_3$, les radicaux phényle, alkyles, et cycloalkyles, les radicaux alkyles droits ou ramifiés ayant de 1 à 4 atomes de carbone, les radicaux cycloalkyles ayant de 3 à 6 atomes de carbone, et leurs sels d'additions aux acides pharmaceutiquement acceptables.

Les composés de l'invention comportent un carbone asymétrique. Les isomères optiquement actifs et les racémates font partie de l'invention.

Les composés préférés de l'invention sont en particulier ceux dans lesquels Ar est le radical phényle ou un radical phényle portant un, deux ou trois substituants choisis parmi les atomes d'halogène et les radicaux méthyle, isopropyle, cyclohexyle et phényle.

Selon l'invention, on prépare les composés (I) par reaction entre une thio-urée de formule (II):

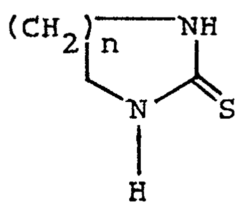

(II)

et une cétone appropriée de formule (III)

$$X—CH_2—CO—CH_2O—Ar \qquad (III)$$

dans laquelle X est un halogène, de préférence le chlore ou le brome, ou tout autre groupe nucléofuge.

Cette réaction est effectuée avantageusement en solution, par exemple, dans de l'acétone, à la température ambiante.

Les cétones (III) sont préparées selon des méthodes connues, déjà décrites, par exemple dans Organic Syntheses Coll., vol. 3, p. 119 et dans les brevets suisses 235 943 et 235 944 et dans le brevet français 6691 M.

On peut utiliser les cétones (III) telles qu'obtenues, sans purification, pour la préparation des composés (I).

Les exemples suivants illustrent l'invention.

Les analyses et spectres IR et RMN confirment la structure des composés.

## Exemple 1

Chlorhydrate d'hydroxy-3[(méthyl-2phénoxy)méthyl]-3 tétrahydro-2,3,5,6 imidazo [2,1-b] thiazole.
[n = 1, Ar = 2-$CH_3$—$C_6H_4$]

Dans un Erlenmeyer der 1 litre, on introduit 6,5 g (0,0636 mole) d'éthylène-thiourée dans 800 $cm^3$ d'acétone et 79,4 g de (méthyl-2 phénoxy)-méthyl chlorométhyl cétone dans 100 $cm^3$ d'acétone. On agite à la température ambiante pendant 20 h. Un solide crème apparaît très rapidement. On le filtre, le rince à l'acétone puis à l'éther.

On recristallise le solide dans un mélange n-propanol/acétone (2/1). F = 157°—158°C.

Exemple 2

Chlorydrate d'hydroxy-3[(dichloro-2,4 phénoxy)méthyl]-3 dihydro-6,7[5H]thiazolo[3,2a]pyrimidine.

[n = 2, Ar = 2,4-di Cl—$C_6H_3$]

Dans un Erlenmeyer de 2 litres, on introduit 5,8 g (0,05 mole) de 3,4,5,6-tétrahydro-pyrimidine-thiol en solution dans 1200 $cm^3$ d'acétone et 44 g (0,173 mole) de (dichloro-2,4 phénoxy)-méthyl chloro-méthyl cétone dans 80 $cm^3$ d'acétone. On agite à la température ambiante pendant 4 h, puis on laisse au repos 48 h. On récupère un solide beige clair que l'on recristallise dans de l'acétone. On obtient un solide blanc.

F = 183°C.

Dans le tableau suivant sont représentés les composés (I) préparés à titre d'exemples.

TABLEAU

| Composé | n | Ar | Sel | F (°C) |
|---|---|---|---|---|
| 1 | 1 | $C_6H_5$ | HCl | 127–8 |
| 2 | 1 | 4–Cl–$C_6H_4$ | HCl | 143,5–144,5 |
| 3 (ex 1) | 1 | 2–$CH_3$–$C_6H_4$ | HCl | 157–8 |
| 4 | 1 | 3–$CF_3$–$C_6H_4$ | HCl | 146–7 |
| 5 | 1 | 3,4–$Cl_2$–$C_6H_3$ | HCl | 153–4 |
| 6 | 1 | 2,6–$(CH_3)_2$–$C_6H_3$ | HCl | 190–1 |
| 7 | 1 | 4–$C_6H_5$–$C_6H_4$ | HCl | 194–5 |
| 8 | 1 | 3–F–$C_6H_4$ | HCl | 144–5 |
| 9 | 1 | 4–Br–$C_6H_4$ | HCl | 156–7 |
| 10 | 1 | 3–Cl–$C_6H_4$ | HCl | 154–155 |
| 11 | 1 | 2-i-$C_3H_7$–$C_6H_4$ | HCl | 134–135,5 |
| 12 | 1 | 2,6–$Cl_2$–$C_6H_3$ | HCl | 163* |
| 13 | 1 | 2–$C_6H_{11}$–$C_6H_4$ (cyclohexyl) | HCl | 185* |
| 14 | 1 | 2–$C_6H_5$–$C_6H_4$ | HCl | 169–170 |
| 15 | 1 | 2,5–$Cl_2$–$C_6H_3$ | HCl | 150* |
| 16 | 1 | 2,4,5–$Cl_3$–$C_6H_2$ | HCl | 165* |
| 17 | 1 | 2–Cl–$C_6H_4$ | HCl | 147–148 |
| 18 | 1 | 2–Br–$C_6H_4$ | HCl | 148–149 |
| 19 | 1 | 2,4–$Cl_2$–$C_6H_3$ | HCl | 155* |
| 20 (ex 2) | 2 | 2,4–$Cl_2$–$C_6H_3$ | HCl | 183* |
| 21 | 2 | 4–$C_6H_5$–$C_6H_4$ | HCl | 193–5 |
| 22 | 2 | 4–Br–$C_6H_4$ | HCl | 192–4 |
| 23 | 2 | 2–$C_6H_5$–$C_6H_4$ | HCl | 188–190 |

*point de fusion déterminé par analyse thermique différentielle.

Les autres points de fusion ont été déterminés au Tottoli.

Les composés de l'invention (I) ont été soumis à des essais pharmacologiques qui ont révélé leur activité antidépressive.

La toxicité des composés a été déterminée chez la souris par voie i.p. La DL 50 varie de 100 à plus de 1000 mg/kg.

L'activité antidépressive a été déterminée selon le test de l'antagonisme de la ptose réserpinique (Gouret C. et coll., J. J. Pharmacol. (Paris) *8*, 333—350 (1977).

Les souris (mâles, CDI Charles River, France, 18—22 g) reçoivent simultanément les produits à étudier ou le solvant *(voie i.p.)*, et la réserpine (4 mg/kg, *voie s.c.)*.

Soixante minutes plus tard, le degré de ptose palpébrale est estimé au moyen d'une échelle de cotation (0 à 4), pour chaque souris.

A chaque dose, la moyenne de cotation et le pourcentage de variation par rapport au lot contrôle, sont calculés.

Pour chaque produit, la DA 50, ou dose qui diminue de 50% le score moyen de ptose par rapport aux contrôles est déterminée graphiquement.

La DA 50 varie de 3 à 10 mg/kg par voie i.p.

Les résultats des tests montrent que les composés de l'invention sont des antidépresseurs utiles pour le traitement de la dépression.

Les composés de l'invention peuvent être présentés sous toute forme appropriée pour l'administration par voie orale, parentérale, endorectale, par exemple sous forme de comprimés, de dragées, de gélules, de solutions buvables ou injectables, etc., avec tout excipient approprié.

La posologie quotidienne peut aller de 5 à 200 mg.

**Revendications pour les États contractants: BE, CH, DE, FR, GB, IT, LU, NL, SE**

1. Dérivés hydroxylés de thiazole, sous la forme de racémates ou d'isomères optiquement actifs, répondant à la formule:

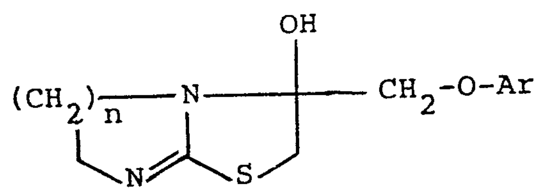

(I)

dans laquelle n est 1 ou 2 et Ar est un radical phényle pouvant porter un ou plusieurs substituants choisis dans le groupe constitué par les atomes d'halogène, $CF_3$, les radicaux phényle, alkyles, et cycloalkyles, les radicaux alkyles droits ou ramifiés ayant de 1 à 4 atomes de carbone, les radicaux cycloalkyles ayant de 3 à 6 atomes de carbone, ainsi que leurs sels d'addition aux acides pharmaceutiquement acceptables.

2. Dérivés selon la revendication 1, dans lesquels Ar est le radical phényle ou un radical phényle portant un, deux ou trois substituants choisis parmi les atomes d'halogène et les radicaux méthyle, isopropyle, cyclohexyle et phényle.

3. L'hydroxy-3 phénoxyméthyl-3 tétrahydro-2,3,5,6 imidazolo[2,1-b]thiazole et son chlorhydrate.

4. Procédé de préparation des dérivés selon la revendication 1, procédé caractérisé en ce que l'on fait réagir une thio-urée de formule (II)

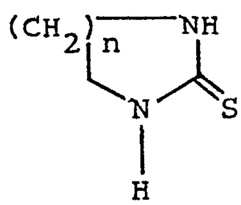

(II)

et une cétone appropriée de formule (III)

$$X—CH_2—CO—CH_2O—Ar \qquad (III)$$

dans laquelle X est un halogène, de préférence le chlore ou le brome, ou tout autre groupe nucléofuge.

5. Médicament caractérisé en ce qu'il contient un composé spécifié dans l'une quelconque des revendications 1 à 3.

**Patentansprüche für die Vertragsstaaten: BE, CH, DE, FR, GB, IT, LU, NL, SE**

1. Hydroxy-thiazol-Derivate, in Form der Racemate oder der optisch aktiven Isomeren, der allgemeinen Formel:

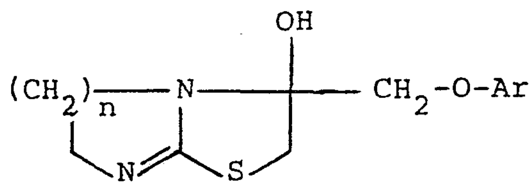
(I)

worin n 1 oder 2 und Ar eine Phenylgruppe, die einen oder mehrere Substituenten aus der Halogenatome, $CF_3$-Gruppen, Phenylgruppen, Alkylgruppen und Cycloalkyl-gruppen umfassenden Gruppe tragen kann, bedeuten, wobei die geradkettigen oder verzweigten Alkylgruppen 1 bis 4 Kohlenstoffatome und die Cycloalkylkgruppen 3 bis 6 Kohlenstoffatome aufweisen, sowie deren Additionssalze mit pharmazeutisch annehmbaren Säuren.

2. Derivate nach Anspruch 1, dadurch gekennzeichnet, daß Ar eine Phenylgruppe oder eine Phenylgruppe, die einen, zwei oder drei Substituenten aus der Halogenatome, Methylgruppen, Isopropylgruppen, Cyclohexylgruppen und Phenylgruppen umfassenden Gruppe trägt, bedeutet.

3. 3-Hydroxy-3-phenoxymethyl-2,3,5,6-tetrahydro-imidazolo[2,1-b]thiazol und dessen Hydrochlorid.

4. Verhfahren zur Herstellung der Verbindungen nach Anspruch 1, dadurch gekennzeichnet, daß man einen Thioharnstoff der Formel II

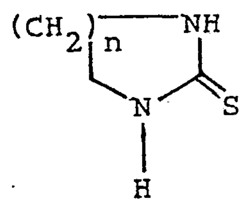
(II)

mit einem geeigneten Keton der Formel III

$$X—CH_2—CO—CH_2O—Ar \qquad (III)$$

worin X ein Halogenatom, vorzugsweise ein Chloratom oder ein Bromatom, oder irgendeine andere nucleofuge Gruppe bedeutet, umsetzt.

5. Arzneimittle, dadurch gekennzeichnet, daß es eine Verbindung nach einem der Ansprüche 1 bis 3 enthält.

**Claims for the Contracting States: BE, CH, DE, FR, GB, IT, LU, NL, SE**

1. Thiazole hydroxyl derivatives, in the form of racemates or optically active isomers, of the formula (I)

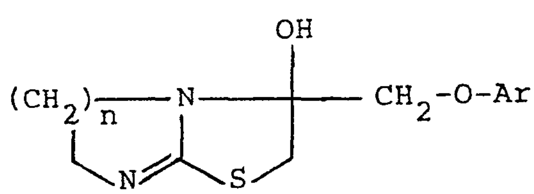
(I)

in which n is 1 or 2 and Ar is a phenyl radical which may be substituted by one or more halogen atoms, $CF_3$ groups, phenyl, alkyl or cycloalkyl radicals, the linear or branched alkyl radicals having from 1 to 4 carbon atoms and the cycloalkyl radicals having from 3 to 6 carbon atoms, and their addition salts with pharmaceutically acceptable acids.

2. Derivatives according to Claim 1, in which Ar is the phenyl radical or a phenyl radical substituted by one or two or three halogen atoms, methyl, isopropyl, cyclohexyl and phenyl radicals.

3. 3-Hydroxy-3-phenoxymethyl-2,3,5,6-tetrahydroimidazolo[2,1-b]thiazole and its hydrochloride.

4. Process for the preparation of the derivatives according to Claim 1, which comprises reacting a thiourea of the formula (II)

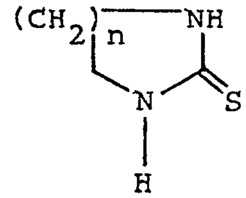

(II)

which an appropriate ketone of the formula (III)

$$X—CH_2—CO—CH_2O—Ar \qquad (III)$$

in which X is a halogen, preferably chlorine or bromine, or any other nucleofugic group.

5. Medicament, characterised in that it contains a compound specified in any one of Claims 1 to 3.

**Revendication pour l'état contractant: AT**

Procédé de préparation de dérivés hydroxylés de thiazole, sous la forme de racémates ou d'isomères optiquement actifs, répondant à la formula:

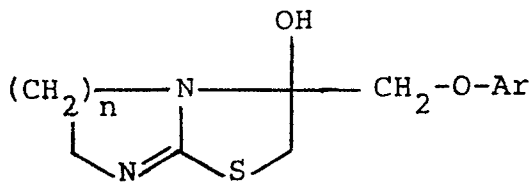

(I)

dans laquelle n est 1 ou 2 et Ar est un radical phényle pouvant porter un ou plusieurs substituants choisis dans le groupe constitué par les atomes d'halogènes, $CF_3$, les radicaux phényle, alkyles, et cycloalkyles, les radicaux alkyles droits ou ramifiés ayant de 1 à 4 atomes de carbone, les radicaux cycloalkyles ayant de 3 à 6 atomes de carbone, ainsi que leurs sels d'addition aux acides pharmaceutiquement acceptables, procédé charactérisé en ce que l'on fait réagir une thio-urée de formule II:

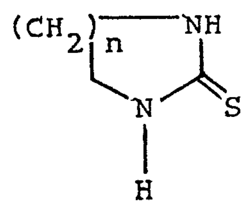

(II)

et une cétone appropriée de formule (III)

$$X—CH_2—CO—CH_2O—Ar \qquad (III)$$

dans laquelle X est un halogène, de préférence le chlore ou le brome, ou tout autre groupe nucléofuge.

**Patentansprüch für den Vertragsstaat: AT**

Verfahren zur Herstellung von Hydroxy-Thiazol-Derivaten, in Form der Racemate oder der optisch aktiven Isomeren, der allgemeinen Formel

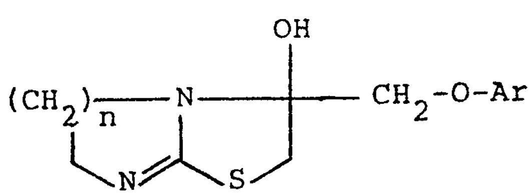

(I)

worin n 1 oder 2 und Ar eine Phenylgruppe, die einen oder mehrere Substituenten aus der Halogenatome, $CF_3$-Gruppen, Phenylgruppen, Alkylgruppen und Cycloalkylgruppen umfassenden Gruppe tragen kann, bedeuten, wobei die geradkettigen oder verzweigten Alkylgruppen 1 bis 4 Kohlenstoffatome und die Cycloalkylgruppen 3 bis 6 Kohlenstoffatome aufweisen, sowie deren Additionssalze mit pharmazeutisch annehmbaren Säuren, dadurch gekennzeichnet, daß man einen Thioharnstoff der Formel II

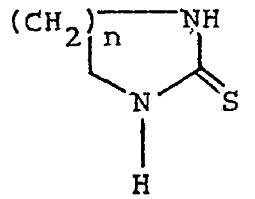

(II)

mit einem geeigneten Keton der Formel III

$$X—CH_2—CO—CH_2O—Ar \qquad (III)$$

worin X ein Halogenatom, vorzugsweise ein Chloratom oder ein Bromatom, oder eine irgendeine andere nucleofuge Gruppe bedeutet, umsetzt.

**Claim for the Contracting State: AT**

Process for the preparation or thiazole hydroxyl derivatives, in the form of racemates or optically active isomers of the formula

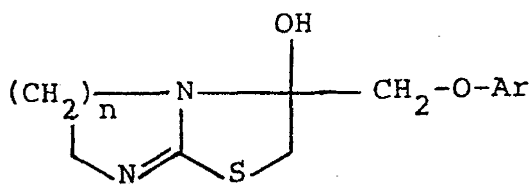

(I)

in which n is 1 or 2 and Ar is a phenyl radical which may be substituted by one or more halogen atoms, $CF_3$ groups, phenyl, alkyl and cycloalkyl radicals, the linear or branched alkyl radicals having from 1 to 4 carbon atoms and the cycloalkyl radicals having from 3 to 6 carbon atoms, and of their addition salts with pharmaceutically acceptable acids, which process comprises reacting a thiourea of the formula II:

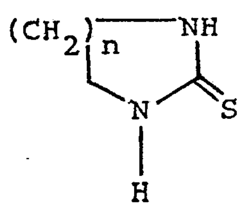

(II)

with an appropriate ketone of the formula (III)

$$X—CH_2—CO—CH_2O—Ar \qquad (III)$$

in which X is a halogen, preferably chlorine or bromine, or any other nucleofugic group.